# EUROPEAN PATENT APPLICATION

(11) **EP 2 048 138 A1**
(43) Date of publication of application: **15.04.2009**
(21) Application number: 07791048.7
(22) Date of filing: 20.07.2007
(51) Int. Cl.: C07D 295/08, A61K 31/495, A61P 3/10, A61P 9/00, A61P 9/04, A61P 9/06, A61P 9/10, A61P 9/12, A61P 21/00, A61P 25/00

(54) **SALT OR SOLVATE OF 5-METHYL-2-(PIPERAZIN-1-YL)BENZENESULFONIC ACID**

(30) Priority: 21.07.2006 JP 2006200071
(71) Applicant: Mitsubishi Tanabe Pharma Corporation, Chuo-ku Osaka-shi Osaka 541-8505 (JP)
(72) Inventor: MASUDA, Katsuhiko, Chuo-ku, Osaka-shi, Osaka 541-8505 (JP)
(74) Representative: HOFFMANN EITLE
(86) International application number: PCT/JP2007/064310
(87) International publication number: WO 2008/010567

(57) **Abstract**

The present invention provides a novel salt form or solvate of 5-methyl-2-(piperazin-1-yl)benzenesulfonic acid.

The present invention provides 5-methyl-2-(piperazin-1-yl)benzenesulfonic acid hydrochloride, calcium 5-methyl-2-(piperazin-1-yl)benzenesulfonate and 5-methyl-2-(piperazin-1-yl)benzenesulfonic acid 2,2,2-tetrafluoroethanolate.

## Description

### Technical Field

The present invention relates to a novel salt of 5-methyl-2-(piperazin-1-yl)benzenesulfonic acid and a pharmaceutical agent comprising the same as an active ingredient. The present invention relates to a novel solvate of 5-methyl-2-(piperazin-1-yl)benzenesulfonic acid and a pharmaceutical agent comprising the same as an active ingredient.

### Background Art

Generally, a crystallized form of a free form and a salt form obtained by crystallization in the co-presence of a counter ion are different in the crystal structures, and due to such difference, various different properties of, for example, ionic nature, solubility, stability, flowability, productivity and the like are expected to be achieved. However, whether a crystal in a salt form, which is different from a free form crystal, can be produced greatly depends on the properties of a target substance, and it is not until various production methods are actually examined that whether a particular substance forms a salt can be clarified. In some cases, for example, even when various combinations of a variety of factors such as the type of counter ion, solvent for crystallization, temperature control, concentration adjustment and the like are examined, a salt form of a particular substance may not be obtained. Naturally, it is not possible to determine whether the obtained crystal of a salt form has ensured quality as a pharmaceutical product unless various properties of the salt form are measured.

It is known that an aminobenzenesulfonic acid derivative represented by the following formula (I)

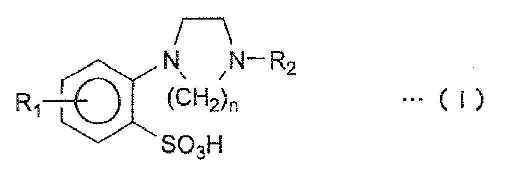

wherein R₁ is a hydrogen atom, a C₁-C₆ alkyl group, a C₃-C₇ cycloalkyl group, a C₁-C₄ halogenated alkyl group, a halogen atom, or a C₆-C₁₂ aryl group; R₂ is a hydrogen atom, a C₁-C₆ alkyl group or a C₇-C₁₂ aralkyl group optionally having one or more substituents selected from the group consisting of a cyano group, a nitro group, a C₁-C₆ alkoxy group, a halogen atom, a C₁-C₆ alkyl group and an amino group; and n is an integer of 1 to 4,
or a salt thereof or a hydrate or solvate thereof suppresses excessive accumulation of intracellular calcium ions in cardiac muscle and blood vessel smooth muscle (patent reference 1). Of such compounds, 5-methyl-2-(piperazin-1-yl)benzenesulfonic acid represented by the following formula (II)

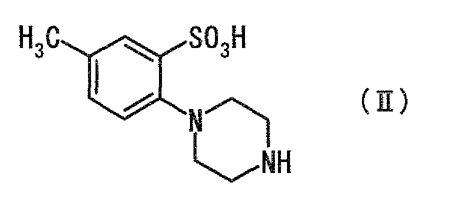

(indicated as 2-(1-piperazinyl)-5-methylbenzenesulfonic acid in patent reference 1; a product of compound No. 12 disclosed in Example of patent reference 1; hereinafter sometimes to be referred to as "compound A") remarkably suppresses excessive influx of calcium ions into cardiomyocytes and shows high safety. Accordingly, it is expected to be extremely useful as an active ingredient of therapeutic agents and/or prophylactic agents for cardiac diseases.

However, no specific description has been made as to the type of salt 5-methyl-2-(piperazin-1-yl)benzenesulfonic acid forms. Furthermore, no specific description has been made as to what kind of solvate is formed other than 5-ethyl-2-(piperazin-1-yl)benzenesulfonic acid monohydrate.

Patent reference 1: JP-A-3-7263, EP-A-390654
Patent reference 2: JP-A-9-221479, EP-A-0779283

### Disclosure of the Invention

### Problems to be Solved by the Invention

An object of the present invention is to provide a novel salt form of 5-methyl-2-(piperazin-1-yl)benzenesulfonic acid.

An object of the present invention is to provide a novel solvate of 5-methyl-2-(piperazin-1-yl)benzenesulfonic acid.

### Means of Solving the Problems

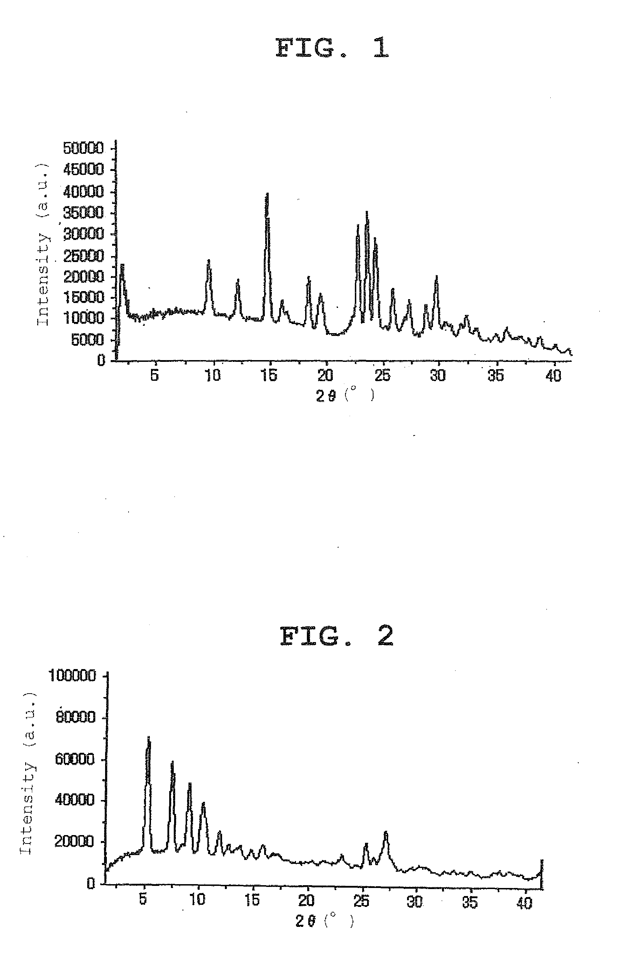

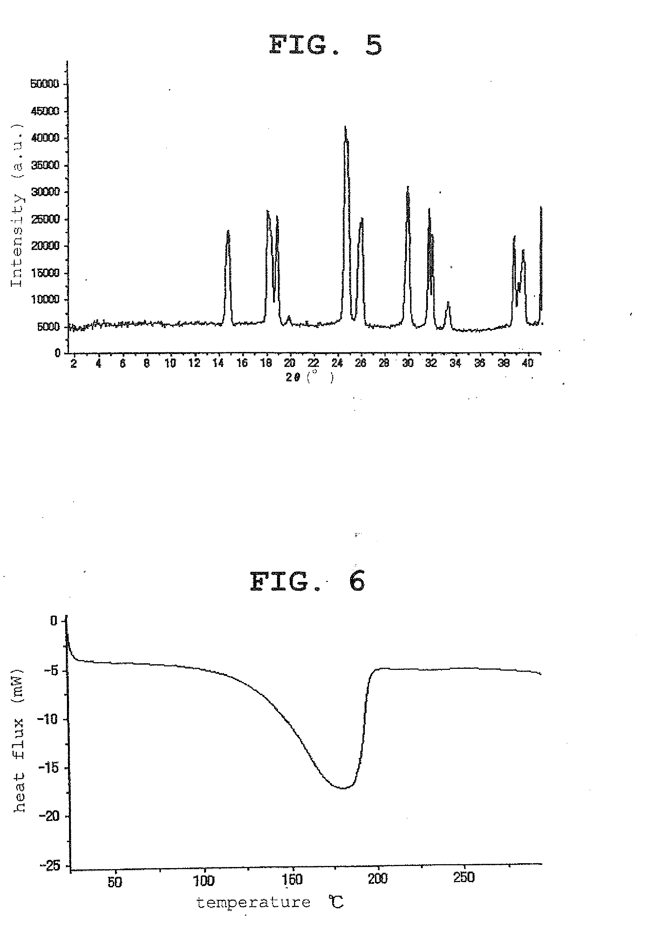

The present inventors have conducted intensive studies in an attempt to solve the above-mentioned problems and found a salt form and a solvate of 5-methyl-2-(piperazin-1-yl)benzenesulfonic acid, which resulted in the completion of the present invention.
Accordingly, the present invention relates to the following.
(1) 5-Methyl-2-(piperazin-1-yl)benzenesulfonic acid hydrochloride.
(2) The 5-methyl-2-(piperazin-1-yl)benzenesulfonic acid hydrochloride of the aforementioned (1), showing diffraction peaks at Bragg angles (2θ) of 9.6°, 12.1°, 14.8°, 22.7° and 23.5° (each ±0.2°) in a powder X-ray diffraction pattern.
(3) 5-Methyl-2-(piperazin-1-yl)benzenesulfonic acid hydrochloride having substantially the same powder X-ray diffraction pattern as that showing Bragg angles (2θ) in the powder X-ray diffraction pattern of Fig. 1.
(4) Calcium 5-methyl-2-(piperazin-1-yl)benzenesulfonate.
(5) The calcium 5-methyl-2-(piperazin-1-yl)benzenesulfonate of the aforementioned (4), showing diffraction peaks at Bragg angles (2θ) of 5.3°, 7.5°, 9.1°, 10.3° and 27.1° (each ±0.2°) in a powder X-ray diffraction pattern.
(6) Calcium 5-methyl-2-(piperazin-1-yl)benzenesulfonate having substantially the same powder X-ray diffraction pattern as that showing Bragg angles (2θ) in the powder X-ray diffraction pattern of Fig. 2.
(7) 5-Methyl-2-(piperazin-1-yl)benzenesulfonic acid 2,2,2-tetrafluoroethanolate.
(8) The 5-methyl-2-(piperazin-1-yl)benzenesulfonic acid 2,2,2-tetrafluoroethanolate of the aforementioned (7), showing diffraction peaks at Bragg angles (2θ) of 14.8°, 18.9°, 24.7°, 30.0° and 31.8° (each ±0.2°) in a powder X-ray diffraction pattern.
(9) 5-Methyl-2-(piperazin-1-yl)benzenesulfonic acid 2,2,2-tetrafluoroethanolate having substantially the same powder X-ray diffraction pattern as that showing Bragg angles (2θ) in the powder X-ray diffraction pattern of Fig. 5.
(10) A drug substance comprising a salt or solvate of the 5-methyl-2-(piperazin-1-yl)benzenesulfonic acid of any of the aforementioned (1) to (9).
(11) A pharmaceutical composition comprising a salt or solvate of the 5-methyl-2-(piperazin-1-yl)benzenesulfonic acid of any of the aforementioned (1) to (9) as an active ingredient.
(12) The pharmaceutical composition of the aforementioned (11), which is used for the prophylaxis and/or treatment of an ischemic cardiac disease or an ischemic circulatory disorder.
(13) The pharmaceutical composition of the aforementioned (11), which is used for the prophylaxis and/or treatment of muscle infarction, angina pectoris, cardiac failure, hypertension, arrhythmia, cardiomyopathy, diastolic disorder, nerve system disorder, cerebrovascular disorder, ischemic cerebrovascular disorder, or cardiac failure or arrhythmia and the like in ischemic cardiac disease derived from diabetes.
(14) The pharmaceutical composition of the aforementioned (11) which is used for the prophylaxis and/or treatment of a circulatory disease by administration to a patient undergoing percutaneous coronary intervention.

### Effect of the Invention

According to the present invention, a hydrochloride salt and a calcium salt of 5-methyl-2-(piperazin-1-yl)benzenesulfonic acid, which are novel salt forms, can be provided.

According to the present invention, a 2,2,2-tetrafluoroethanolate of 5-methyl-2-(piperazin-1-yl)benzenesulfonic acid, which is a novel solvate, can be provided.

The salt and solvate of the present invention do not show weight variation during preservation, and can be stably supplied as active ingredient drugs of pharmaceutical products.

### Brief Description of the Drawings

Fig. 1 shows a powder X-ray diffraction pattern of 5-methyl-2-(piperazin-1-yl)benzenesulfonic acid hydrochloride.
Fig. 2 shows a powder X-ray diffraction pattern of calcium 5-methyl-2-(piperazin-1-yl)benzenesulfonate.
Fig. 3 shows thermal analysis curve of 5-methyl-2-(piperazin-1-yl)benzenesulfonic acid hydrochloride.
Fig. 4 shows thermal analysis curve calcium 5-methyl-2-(piperazin-1-yl)benzenesulfonate.
Fig. 5 shows a powder X-ray diffraction pattern of 5-methyl-2-(piperazin-1-yl)benzenesulfonic acid 2,2,2-tetrafluoroethanolate.
Fig. 6 shows thermal analysis curve of 5-methyl-2-(piperazin-1-yl)benzenesulfonic acid 2,2,2-tetrafluoroethanolate.

### Best Mode for Carrying out the Invention

The "5-methyl-2-(piperazin-1-yl)benzenesulfonic acid" of the present invention is a compound represented by the following structural formula (II), and the novel salt form of the present invention is a hydrochloride salt or a calcium salt of the following compound. In addition, the novel solvate form of the present invention is 2,2,2-tetrafluoroethanolate.

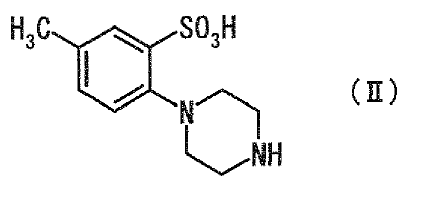

Such salt and solvate each have the physicochemical properties shown in the below-mentioned Examples.

The salt and solvate of the present invention have an action to suppress excessive accumulation of intracellular calcium ions in cardiac muscle or blood vessel smooth muscle, an action to inhibit sodium/calcium exchange system, and an action to suppress excessive accumulation of intracellular sodium ions. Accordingly, a pharmaceutical agent containing the salt of the present invention as an active ingredient is useful for the treatment and/or prophylaxis of circulatory diseases and the like caused by excessive accumulation of intracellular calcium ions and/or excessive accumulation of intracellular sodium ions.

A pharmaceutical agent containing the salt or solvate of the present invention as an active ingredient is effective for the treatment and/or prophylaxis of ischemic cardiac diseases or ischemic circulatory disorders, and preferably, effective for the treatment and/or prophylaxis of myocardial infarction, angina pectoris, cardiac failure, hypertension, arrhythmia, cardiomyopathy, diastolic disorder, nerve system disorder, cerebrovascular disorder, ischemic cerebrovascular disorder, or cardiac failure or arrhythmia in ischemic cardiac diseases derived from diabetes.

A pharmaceutical agent containing the salt or solvate of the present invention as an active ingredient is used for the prophylaxis and/or treatment of circulatory disorders. It is effective as a pharmaceutical agent to be administered to patients undergoing percutaneous coronary intervention, and also effective for the treatment of myocardial infarction and the like in such patients.

The ischemic circulatory disorder in the present invention refers to a disorder in the circulatory organ, which is caused by blockage of the blood vessel due to some reason such as formation of thrombus and the like, and specifically means, for example, myocardial infarction or angina pectoris. The type of myocardial infarction, for which the present invention is effective, is ST segment elevation myocardial infarction (STEMI).

When the salt or solvate of the present invention is administered as a pharmaceutical product, it is applied to human orally or parenterally according to a conventional method. Examples of the dosage form for oral administration include granule, fine granule, powder, tablet, hard capsule, soft capsule, syrup, emulsion, suspension, liquid and the like. In addition, examples of the dosage form for parenteral administration include injection, suppository, transdermal agent and the like.

A pharmaceutical composition comprising the salt or solvate of the present invention as an active ingredient also concurrently contains a solid or liquid carrier for pharmaceutical agents or a pharmaceutical additive to be generally used, such as excipient, stabilizer, lubricant, sweetening agent, preservative, suspending agent and the like. The content ratio of the prophylactic and/or therapeutic active ingredient to the carrier component is preferably within the range of 1 wt% to 90 wt%.

The dose of the salt or solvate of the present invention to be used as an active ingredient can be appropriately determined for each active ingredient in consideration of the object of prophylaxis or treatment, the kind of disease to be prevented or treated, symptom, body weight, age, sex and the like of patients. In general, about 0.001 mg - 100 mg can be administered per day to an adult by parenteral administration, and about 0.01 mg - 1000 mg can be administered by oral administration. Such dose is desirably administered in one to several portions a day.

As a production method of the salt or solvate of the present invention, a method comprising dissolving a compound in a suitable solvent, and adding a suitable amount of an acidic (or basic) compound, an organic solvent and the like to give a solid can be used. In this case, as a method for confirming the formation of a salt or solvate, elemental analysis, single-crystal X-ray structural analysis and the like are generally used. Using elemental analysis, single-crystal X-ray structural analysis and the like for the salt or solvate of the present invention, formation of hydrochloride of 5-methyl-2-(piperazin-1-yl)benzenesulfonic acid, a calcium salt of 5-methyl-2-(piperazin-1-yl)benzenesulfonic acid or 5-methyl-2-(piperazin-1-yl)benzenesulfonic acid 2,2,2-tetrafluoroethanolate can be confirmed.

### Examples

The present invention is explained in more detail in the following by referring to Examples, which are not to be construed as limitative as long as they do not go beyond the scope of the invention.

### (Explanation of measurement device used in Examples and the like)

Each crystal form was confirmed by powder X-ray diffractometer (manufactured by Bruker) GADDS detector system and thermal analysis equipment (manufactured by Mettler-Toledo) DSC822e system.
Powder X-ray diffraction pattern was measured at room temperature using CuKα as a monochromator within the 2θ angle range of 1.5 - 41.5°.
For thermal analysis, the measurement was performed in a dry nitrogen gas stream (50 mL/min) from 25°C to 300°C at a temperature rise rate of 20°C/min.
For identification of the solvate form of the present invention, a powder X-ray diffraction apparatus (manufactured by Bruker) GADDS detector system and a thermal analysis equipment (manufactured by Mettler-Toledo) DSC822e system were used. The powder X-ray diffraction pattern was measured at room temperature using CuKα as a monochromator within the 2θ angle range of 1.5 - 41.5°. The thermal analysis was performed in a dry nitrogen gas stream (50 mL/min) from 25°C to 300°C at a temperature rise rate of 20°C/min.
5-Methyl-2-(piperazin-1-yl)benzenesulfonic acid monohydrate (hereinafter sometimes to be referred to as "caldaret") used in the following Examples was produced according to the method described in patent reference 2, Example 1.

### (Example 1) 5-methyl-2-(piperazin-1-yl)benzenesulfonic acid hydrochloride

5-Methyl-2-(piperazin-1-yl)benzenesulfonic acid monohydrate (75 mg) was added to cyclohexanone (1 mL), hydrochloric acid (1.1-fold molar equivalent relative to 5-methyl-2-(piperazin-1-yl)benzenesulfonic acid monohydrate) was added thereto, and the mixture was stirred at 60°C for 30 min. Thereafter, the mixture was gradually cooled (2°C/hr), and the crystals were matured at 5°C for 72 hr to give a white solid.

### (Example 2) calcium 5-methyl-2-(piperazin-1-yl)benzenesulfonate

5-Methyl-2-(piperazin-1-yl)benzenesulfonic acid monohydrate (75 mg) was added to diisopropyl ether (1 mL), calcium acetate (1.1-fold molar equivalent relative to 5-methyl-2-(1-piperazinyl)benzenesulfonic acid monohydrate) was added thereto, and the mixture was stirred at 60°C for 30 min. Thereafter, the mixture was gradually cooled (2°C/hr), and the crystals were matured at 25°C for 72 hr to give a white solid. (Experimental Example 1) Measurement of powder X-ray diffraction
Using the white solids obtained in Examples 1 and 2 as sample 1 (hydrochloride salt) and sample 2 (calcium salt) respectively, the powder X-ray diffraction was measured under the following conditions.

### Measurement conditions

apparatus: D8 manufactured by Bruker
X-ray source: CuKα
detector: GADDS (Hi-STAR)
measurement range: 1.5 - 41.5°(2θ)
measurement temperature: room temperature
The powder X-ray diffraction patterns of sample 1 (hydrochloride salt) and sample 2 (calcium salt) are shown in Fig. 1 and Fig. 2, respectively.
The characteristic peaks of sample 1 (hydrochloride salt) in terms of diffraction angle represented by 2θ were 9.6, 12.1, 14.8, 22.7 and 23.5° (each +0.2°). The X-ray diffraction pattern was completely different from that of 5-methyl-2-(piperazin-1-yl)benzenesulfonic acid heretofore known, and it was confirmed that the crystal was novel.
The characteristic peaks of sample 2 (calcium salt) in terms of diffraction angle represented by 2θ were 5.3, 7.5, 9.1, 10.3 and 27.1° (each ±0.2°). The X-ray diffraction pattern was completely different from that of 5-methyl-2-(piperazin-1-yl)benzenesulfonic acid heretofore known, and it was confirmed that the crystal was novel.

### (Experimental Example 2) Thermal analysis

Using the white solids obtained in Examples 1 and 2 as sample 1 (hydrochloride salt) and sample 2 (calcium salt), respectively, the thermal analysis was performed under the following conditions.

### Measurement conditions

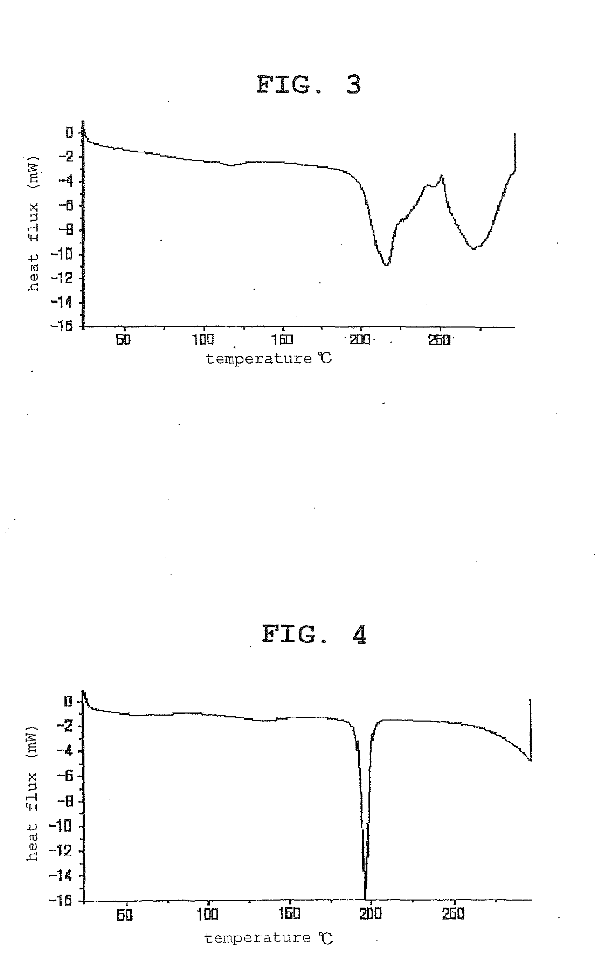

apparatus: DSC822e manufactured by Metter-Toledo
temperature rise rate: 20°C/min
atmosphere: dry nitrogen gas 50 mL/min
measurement temperature: 25 - 300°C
The thermal analysis curves of sample 1 (hydrochloride salt) and sample 2 (calcium salt) are shown in Fig. 3 and Fig. 4, respectively.
Both sample 1 (hydrochloride salt) and sample 2 (calcium salt) did not show noticeable endothermic and exothermic peaks up to 200°C, and it was confirmed that the crystals were stable. Accordingly, the salt of the present invention can be stably supplied as an active ingredient drug of pharmaceutical products.

### (Example 3) 5-methyl-2-(piperazin-1-yl)benzenesulfonic acid 2,2,2-tetrafluoroethanolate.

5-Methyl-2-(piperazin-1-yl)benzenesulfonic acid monohydrate (25 mg) was heated to 80°C with stirring while gradually adding 2,2,2-tetrafluoroethanol (about 1 mL). After confirmation of the suspension state of the system, hot filtration was performed to give a saturated solution at 80°C. Water in the same volume as 2,2,2-tetrafluoroethanol was added to the saturated solution to give a white solid.

### (Experimental Example 3) Measurement of powder X-ray diffraction

Using the solid obtained in Example 3 as sample 3 (2,2,2-tetrafluoroethanol hydrate), the powder X-ray diffraction was measured under the following conditions.

### Measurement conditions

apparatus: D8 manufactured by Bruker
X-ray source : CuKα
detector : GADDS (Hi-STAR)
measurement range : 1.5 - 41.5° (2θ)
measurement temperature : room temperature
The powder X-ray diffraction pattern of sample 3 (2,2,2-tetrafluoroethanol hydrate) is respectively shown in Fig. 5.
The characteristic peaks of sample 3 (2,2,2-tetrafluoroethanol hydrate) in terms of diffraction angle represented by 2θ were 14.8, 18.9, 24.7, 30.0 and 31.8° (each ±0.2°). The X-ray diffraction pattern was completely different from that of 5-methyl-2-(piperazin-1-yl)benzenesulfonic acid heretofore known, and it was confirmed that the crystal was novel.

### (Experimental Example 4) Thermal analysis

Using the solid obtained in Example 3 as sample 3 (2,2,2-tetrafluoroethanol hydrate crystal), the thermal analysis was performed under the following conditions.

### Measurement conditions

apparatus: DSC822e manufactured by Metter-Toledo
temperature rise rate: 20°C/min
atmosphere: dry nitrogen gas 50 mL/min
measurement temperature: 25 - 300°C
The thermal analysis curve of sample 3 (2,2,2-tetrafluoroethanol hydrate crystal) are respectively shown in Fig. 6.
Sample 3 (2,2,2-tetrafluoroethanol hydrate crystal) did not show a noticeable endothermic and exothermic peaks up to around 80°C, and it was confirmed that the crystals were stable. In addition, the heatgram obtained by the thermoanalytical measurement was different from that obtained heretofore, and it was confirmed that the crystal was novel. Accordingly, the novel crystal of the present invention can be stably supplied as an active ingredient drug of pharmaceutical products.

### Industrial Applicability

According to the present invention, a novel salt form of 5-methyl-2-(piperazin-1-yl)benzenesulfonic acid can be provided, which is expected to be effective as an active ingredient of prophylactic or therapeutic agents for cardiac diseases.

According to the present invention, a novel solvate of 5-methyl-2-(piperazin-1-yl)benzenesulfonic acid can be provided, which is expected to be effective as an active ingredient of prophylactic or therapeutic agents for cardiac diseases.

This application is based on a patent application No. 2006-200071 filed in Japan, the contents of which are incorporated in full herein by this reference.

## Claims

1. 5-Methyl-2-(piperazin-1-yl)benzenesulfonic acid hydrochloride.

2. The 5-methyl-2-(piperazin-1-yl)benzenesulfonic acid hydrochloride of claim 1, showing diffraction peaks at Bragg angles (2θ) of 9.6°, 12.1°, 14.8°, 22.7° and 23.5° (each ±0.2°) in a powder X-ray diffraction pattern.

3. 5-Methyl-2-(piperazin-1-yl)benzenesulfonic acid hydrochloride having substantially the same powder X-ray diffraction pattern as that showing Bragg angles (2θ) in the powder X-ray diffraction pattern of Fig. 1.

4. Calcium 5-methyl-2-(piperazin-1-yl)benzenesulfonate.

5. The calcium 5-methyl-2-(piperazin-1-yl)benzenesulfonate of claim 4, showing diffraction peak at Bragg angles (2θ) of 5.3°, 7.5°, 9.1°, 10.3° and 27.1° (each ±0.2°) in a powder X-ray diffraction pattern.

6. Calcium 5-methyl-2-(piperazin-1-yl)benzenesulfonate having substantially the same powder X-ray diffraction pattern as that showing Bragg angles (2θ) in the powder X-ray diffraction pattern of Fig. 2.

7. 5-Methyl-2-(piperazin-1-yl)benzenesulfonic acid 2,2,2-tetrafluoroethanolate.

8. The 5-methyl-2-(piperazin-1-yl)benzenesulfonic acid 2,2,2-tetrafluoroethanolate of claim 7, showing diffraction peaks at Bragg angles (2θ) of 14.8°, 18.9°, 24.7°, 30.0° and 31.8° (each ±0.2°) in a powder X-ray diffraction pattern.

9. 5-Methyl-2-(piperazin-1-yl)benzenesulfonic acid 2,2,2-tetrafluoroethanolate having substantially the same powder X-ray diffraction pattern as that showing Bragg angles (2θ) in the powder X-ray diffraction pattern of Fig. 5.

10. A drug substance comprising a salt or solvate of the 5-methyl-2-(piperazin-1-yl)benzenesulfonic acid of any of claims 1 to 9.

11. A pharmaceutical composition comprising a salt or solvate of the 5-methyl-2-(piperazin-1-yl)benzenesulfonic acid of any of claims 1 to 9 as an active ingredient.

12. The pharmaceutical composition of claim 11, which is used for the prophylaxis and/or treatment of an ischemic cardiac disease or an ischemic circulatory disorder.

13. The pharmaceutical composition of claim 11, which is used for the prophylaxis and/or treatment of muscle infarction, angina pectoris, cardiac failure, hypertension, arrhythmia, cardiomyopathy, diastolic disorder, nerve system disorder, cerebrovascular disorder, ischemic cerebrovascular disorder, or cardiac failure or arrhythmia and the like in ischemic cardiac disease derived from diabetes.

14. The pharmaceutical composition of claim 11, which is used for the prophylaxis and/or treatment of a circulatory disease by administration to a patient undergoing percutaneous coronary intervention.
